# EUROPÄISCHE PATENTSCHRIFT

(11) **EP 2 581 398 B1**
(45) Veröffentlichungstag und Bekanntmachung des Hinweises auf die Patenterteilung: **06.08.2014**
(21) Anmeldenummer: 11185179.6
(22) Anmeldetag: 14.10.2011
(51) Int. Cl.: C08G 18/78, C08G 18/81, C09D 175/16

(54) **Verfahren zur Herstellung von niedrigviskosen, wasseremulgierbaren Allophanaten mit strahlenhärtbaren Gruppen**
Method for producing low viscosity, water-emulsifiable allophanates with radiation curable groups
Procédé de fabrication d'allophanates peu visqueux hydroémulsifiants à l'aide de groupes pouvant être durcis par rayonnement

(43) Veröffentlichungstag der Anmeldung: 17.04.2013
(73) Patentinhaber: Allnex IP S.à.r.l., 1660 Luxembourg (LU)
(72) Erfinder: Dr. Sommer Stefan, Leverkusen (DE); Dr. Ludewig Michael, Leverkusen (DE); Dr. Fischer Wolfgang, Leverkusen (DE); Dr. Weikard Jan, Leverkusen (DE); Lippemeier Jürgen, Leverkusen (DE)
(74) Vertreter: Destryker, Elise Martine

(56) Entgegenhaltungen:
- EP-A1- 2 218 746
- EP-A2- 2 031 005

## Beschreibung

Die vorliegende Erfindung betrifft ein Verfahren zur Herstellung von niedrigviskosen, Allophanatgruppen enthaltenden Umsetzungsprodukten von Polyisocyanaten, die sich leicht in Wasser emulgieren lassen und die aktivierte, unter Einwirkung aktinischer Strahlung mit ethylenisch ungesättigten Verbindungen unter Polymerisation reagierende Gruppen enthalten. Die vorliegende Erfindung betrifft weiterhin die Produkte, die herstellbar sind gemäß dem erfindungsgemäßen Verfahren sowie ihre Verwendung.

Die Härtung von aktivierten Doppelbindungen tragenden Beschichtungssystemen durch aktinische Strahlung ist bekannt und technisch etabliert. Unter aktinischer Strahlung wird elektromagnetische, ionisierende Strahlung verstanden, insbesondere Elektronenstrahlen, UV-Strahlen sowie sichtbares Licht (Roche Lexikon Medizin, 4.Auflage; Urban & Fischer Verlag, München 1999). Es ist eine der schnellsten Härtungsmethoden in der Beschichtungstechnologie. Auf diesem Prinzip basierende Beschichtungsmittel werden daher als strahlen- oder aktinisch-härtende bzw. härtbare Systeme bezeichnet.

Bedingt durch die ökologischen und ökonomischen Anforderungen an moderne Lacksysteme möglichst wenig oder gar kein organisches Lösungsmittel zur Viskositätseinstellung zu verwenden, besteht der Wunsch, einerseits bereits niedrigviskose Lackrohstoffe zu verwenden und anderseits darüber hinaus notwendige Viskositätseinstellungen mit Wasser als Lösemittel durchführen zu können.

Besonders niederviskose, strahlenhärtende Bindemittel sind bekanntermaßen Allophanatgruppen enthaltende Urethanacrylate. Diese können durch verschiedene Verfahren hergestellt werden, so z.B. durch direkte Umsetzung mit einem Überschuss Diisocyanat und anschließender Destillation des Überschusses an Diisocyanat (EP-A 0 867 457 oder WO-A 00/39183), durch Öffnung eines Oxadiazintrions (WO-A 2004/033522), durch Öffnung eines Uretdions (WO-A 2005/092942) oder auch durch direkte Allophanatisierung eines equimolaren Verhältnisses von Isocyanatgruppen zu Urethangruppen (EP-A 1 645 582). Lediglich die letztgenannte Anmeldung offenbart auch die Möglichkeit, über eine Hydrophilierung zu wasseremulgierbaren Bindemitteln zu kommen. Allerdings sind die so erhaltenen Produkte nicht besonders stabil in der Emulsion und es tritt bereits nach kurzer Zeit eine Phasentrennung auf.

Es sind auch schon Allophanatgruppen enthaltende Urethanacrylate bekannt geworden, die durch eingebaute Hydrophilierungsmittel wasseremulgierbar sind. So beschreibt die EP-A 0 694 531 eine Methode zu Herstellung solcher Bindemittel. Allerdings wird hier durch die Wahl einer ionischen Hydrophilierung auf eine Minimierung der Viskosität verzichtet. Weiterhin handelt es sich um ein kompliziertes mehrstufiges Verfahren, das bei sehr hohen Temperaturen von mehr als 100°C durchgeführt werden muss, was der Stabilität von Systemen mit aktivierten Doppelbindungen abträglich ist. Schließlich zielt das Verfahren nicht auf niederviskose 100%-Systeme, es werden bereits fertige Emulsionen erhalten.

In der WO-A 2007/063025 wird ebenfalls ein Allophanatgruppen-haltiges, wasseremulgierbares Urethanacrylat beschrieben. Allerdings werden diese Allophanatstrukturen nicht im offenbarten Verfahren erzeugt, sondern über ein modifiziertes Isocyanat eingebracht. Solche modifizierten Isocyanate sind nur schwer und aufwändig herzustellen, da das in der WO-A 00/39183 beschriebene Verfahren einen technisch nur schwer umsetzbaren Destillationsprozess enthält. Letztlich ist also die Isolation eines Zwischenproduktes nötig.

Es war daher Aufgabe der vorliegenden Erfindung, ein einfaches Verfahren zur Herstellung eines strahlenhärtbaren Polyurethan(meth)acrylats zur Verfügung zu stellen, das, basierend auf Allophanat-Strukturen als unverdünntes System mit einem Festkörpergehalt von 100 Gew.-% besonders niedrigviskos ist und sich leicht durch Einrühren in Wasser emulgieren lässt. Zudem soll die mit Wasser verdünnte strahlenhärtbare Polyurethandispersion lagerstabil sein.

Überraschend wurde ein Verfahren zur Herstellung von wasseremulgierbaren, strahlenhärtenden Allophanaten mit Restmonomergehalten von weniger als 0,5 Gew.-% und einem NCO-Gehalt von weniger als 1 Gew.-% gefunden, bei dem aus
A) mindestens einer isocyanatgruppenhaltigen Verbindung,
B) mindestens einer hydroxyfunktionellen Verbindung, die unter Einwirkung aktinischer Strahlung mit ethylenisch ungesättigten Verbindungen unter Polymerisation reagierende Gruppen (strahlenhärtende Gruppen) aufweist,
C) mindestens einem Polyoxyalkylenmono-ol
D) gegebenenfalls mindestens eine von B) und/oder C) verschiedene Verbindung mit NCO-reaktiven Gruppen
E) gegebenenfalls in Anwesenheit eines Katalysators
   NCO-gruppenhaltige Urethane mit strahlenhärtenden Gruppen gebildet werden die anschließend ohne weitere Zugabe an isocyanatgruppenhaltigen Verbindungen in Anwesenheit
F) eines Allophanatisierungskatalysators und
G) gegebenenfalls eines tertiäten Amins
umgesetzt werden,
wobei das Verhältnis von NCO-Gruppen der Verbindungen aus A) zu den OH-Gruppen von den Verbindungen aus B) und C) und ggf. D) 1,80 : 1,0 bis 1,46 : 1,0, bevorzugt 1,70 : 1,0 bis 1,47 : 1,0, besonders bevorzugt 1,65 : 1,0 bis 1,48 : 1,0 und ganz besonders bevorzugt 1,60 : 1,0 bis 1,50 : 1,0 beträgt.

Der Begriff "wasseremulgierbar" im Sinne der Erfindung bedeutet, dass sich die erfindungsgemäßen Polyurethane mit Wasser mischen lassen und sich dabei über einen weiten Bereich der Mischungsverhältnisse eine Emulsion bildet. Der Festkörpergehalt von 100 Gew.-% bedeutet, dass das Polyurethansystem nicht mit Wasser verdünnt wurde.

Ein weiterer Gegenstand der Erfindung sind wasseremulgierbare Urethan(meth)acrylate erhältlich nach dem erfindungsgemäßen Verfahren.

Ein weiterer Gegenstand der Erfindung ist die Verwendung der wasseremulgierbaren Urethan(meth)acrylate, erhältlich nach dem erfindungsgemäßen Verfahren, für die Herstellung von Beschichtungen und Lacken sowie Klebstoffen, Druckfarben, Gießharzen, Dentalmassen, Schlichten, Photoresisten, Stereolithographiesystemen, Harzen für Verbundwerkstoffe und Dichtungsmassen.

"(Meth)acrylat" bezieht sich im Rahmen dieser Erfindung auf entsprechende Acrylat- oder Methacrylatfunktionen oder auf eine Mischung beider.

Als isocyanathaltige Verbindungen A) kommen aromatische, aliphatische und cycloaliphatische Polyisocyanate in Betracht. Geeignete Polyisocyanate sind Verbindungen der Formel Q(NCO)ₙ mit einem mittleren Molekulargewicht unter 800, worin n eine Zahl von 2 bis 4 und Q einen aromatischen C₆-C₁₅-Kohlenwasserstoffrest, einen aliphatischen C₄-C₁₂-Kohlenwasserstoffrest oder einen cycloaliphatischen C₆-C₁₅-Kohlenwasserstoffrest bedeuten, beispielsweise Diisocyanate aus der Reihe 2,4-/2,6-Toluoldiisocyanat (TDI), Methylendiphenyldiisocyanat (MDI), Triisocyanatononan (TIN), Naphtyldiisocyanat (NDI), 4,4'-Diiso-cyanatodicyclohexylmethan, 3-Isocyanatomethyl-3,3,5-trimethylcyclohexylisocyanat (Isophorondiisocyanat = IPDI), Tetramethylendiisocyanat, Hexamethylendiisocyanat (HDI), 2-Methyl-pentamethylendiisocyanat, 2,2,4-Trimethylhexamethylendiisocyanat (THDI), Dodecamethylendiisocyanat, 1,4-Diisocyanato-cyclohexan, 4,4'-Diisocyanato-3,3'-dimethyl-dicyclohexylmethan, 4,4'-Diisocyanatodicyclohexylpropan-(2,2), 3-Isocyanatomethyl-1-methyl-1-isocyanatocyclohexan (MCI), 1,3-Diisooctylcyanato-4-methyl-cyclohexan, 1,3-Diisocyanato-2-methyl-cyclohexan und α,α,α',α'-Tetramethyl-m- oder -p-xylylen-diisocyanat (TMXDI) sowie aus diesen Verbindungen bestehende Gemische.

Ebenfalls geeignet als isocyanathaltige Verbindungen A) sind Umsetzungsprodukte der vorgenannten Isocyanate mit sich selbst oder untereinander zu Polyisocyanaten mit Uretdion-, Isocyanaurat-, Allophanat-, Biuret-, Iminooxadiazindion- und/oder Oxadiazintrionstruktur, wie sie beispielsweise in J. Prakt. Chem. 336 (1994) 185 - 200 und EP-A 0 798 299 beispielhaft beschrieben sind.

Weiterhin geeignet als isocyanathaltige Verbindungen A) sind Umsetzungsprodukte der vorgenannten Isocyanate mit anderen isocyanatreaktiven Verbindungen zu Prepolymeren. Solche isocyanatreaktiven Verbindungen sind vor allem Polyole, wie z.B. Polyetherpolyole, Polyesterpolyole, Polycarbonatpolyole und mehrwertige Alkohole. Als Polyole können höhermolekulare und in untergeordneter Menge auch niedermolekulare Hydroxylverbindungen eingesetzt werden.

Die Verbindungen der Komponente A) können dementsprechend direkt in das erfindungsgemäße Verfahren eingesetzt werden oder, ausgehend von einer beliebigen Vorstufe durch Vorreaktion hergestellt werden, bevor das erfindungsgemäße Verfahren durchgeführt wird.

Bevorzugt als Komponente A) ist die Verwendung von monomeren Diisocyanaten. Besonders bevorzugt ist die Verwendung von Hexamethylendiisocyanat (HDI), Isophorondiisocyanat (IPDI) und/oder 4,4'-Diisocyanatodicyclohexylmethan. Ganz besonders bevorzugt ist die Verwendung von Hexamethylendiisocyanat.

Unter aktinischer Strahlung wird elektromagnetische, ionisierende Strahlung verstanden, insbesondere Elektronenstrahlen, UV-Strahlen sowie sichtbares Licht (Roche Lexikon Medizin, 4.Auflage; Urban & Fischer Verlag, München 1999).

Unter Einwirkung aktinischer Strahlung mit ethylenisch ungesättigten Verbindungen unter Polymerisation reagierende Gruppen (strahlenhärtende Gruppen) werden im Rahmen der vorliegenden Erfindung Vinylether-, Maleinyl-, Fumaryl-, Maleinimid-, Dicyclopentadienyl- , Acrylamid-, Acryl- und Methacryl-Gruppen verstanden, wobei dies bevorzugt Vinylether-, Acrylat- und/oder Methacrylat-Gruppen, besonders bevorzugt Acrylatgruppen sind.

Beispielsweise geeignete hydroxylgruppenhaltige Verbindungen der Komponente B) sind 2-Hydroxyethyl(meth)acrylat, Polyethylenoxid-mono(meth)acrylat (z.B. PEA6 / PEM6; Laporte Performance Chemicals Ltd., UK), Polypropylenoxidmono(meth)acrylat (z.B. PPA6, PPM5S; Laporte Performance Chemicals Ltd., UK), Polyalkylenoxid-mono(meth)acrylat (z.B. PEM63P, Laporte Performance Chemicals Ltd. , UK), Poly(ε-caprolacton)mono(meth)acrylate wie z.B. Tone M100^{®} (Dow, Schwalbach, DE), 2-Hydroxypropyl(meth)acrylat, 4-Hydroxybutyl(meth)acrylat, Hydroxybutylvinylether, 3-Hydroxy-2,2-dimethylpropyl(meth)acrylat, die hydroxyfunktionellen Mono-, Di- oder soweit möglich höheren Acrylate wie z.B. Glycerindi(meth)acrylat, Trimethylolpropandi(meth)acrylat, Pentaerythrittri(meth)acrylat oder Dipentaerythritpenta-(meth)acrylat, die durch Umsetzung mehrwertiger gegebenenfalls alkoxylierter Alkohole wie Trimethylolpropan, Glycerin, Pentaerythrit, Dipentaerythrit zugänglich sind.

Ebenfalls geeignet als Bestandteil von B) sind auch Alkohole, die sich aus der Umsetzung von doppelbindungshaltigen Säuren mit gegebenenfalls doppelbindungshaltigen Epoxidverbindungen erhalten werden, so z.B. die Umsetzungsprodukte von (Meth)acrylsäure mit Glycidyl(meth)acrylat oder Bisphenol-A-diglycidylether.

Darüber hinaus können ebenfalls ungesättigte Alkohole eingesetzt werden, die aus der Umsetzung von gegebenenfalls ungesättigten Säureanhydriden mit gegebenenfalls acrylatgruppenhaltigen Hydroxy- und Epoxidverbindungen erhalten werden. Beispielsweise sind dies die Umsetzungsprodukte von Maleinsäureanhydrid mit 2-Hydroxyethyl(meth)acrylat und Glycidyl(meth)acrylat.

Besonders bevorzugt entsprechen die Verbindungen der Komponente B) der vorgenannten Art und weisen eine OH-Funktionalität von 0,9 bis 1,1 auf.

Bevorzugt ist die Verwendung von Hydroxyethyl(meth)acrylat, Hydroxypropyl(meth)acrylat und/oder Hydroxybutyl(meth)acrylat. Ganz besonders bevorzugt sind Hydroxyethylacrylat und/oder Hydroxypropylacrylat.

Polyoxyalkylenmono-ole C) werden erfindungsgemäß als nichtionisch hydrophilierend wirkende Verbindungen eingesetzt. Diese Polyoxyalkylenmono-ole enthalten einen Anteil von 30 Gew.-% bis 100 Gew.-% an Bausteinen, die vom Ethylenoxid abgeleitet sind.

Vorzugsweise werden im statistischen Mittel 5 bis 70, bevorzugt 7 bis 55 Ethylenoxideinheiten pro Molekül aufweisende Polyoxyalkylenmono-ole eingesetzt, wie sie in an sich bekannter Weise durch Alkoxylierung geeigneter Startermoleküle zugänglich sind (z.B. in Ullmanns Encyclopädie der technischen Chemie, 4 .Auflage, Band 19, Verlag Chemie, Weinheim S. 31-38).

Geeignete Startermoleküle zur Herstellung dieser Polyoxyalkylenethermono-ole sind beispielsweise gesättigte Monoalkohole wie Methanol, Ethanol, n-Propanol, Isopropanol, n-Butanol, Isobutanol, sec-Butanol, die Isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykol-monoalkylether wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole, besonders bevorzugte Startermoleküle sind Methanol oder Ethanol.

Für die Alkoxylierungsreaktion geeignete Alkylenoxide sind beispielsweise Ethylenoxid, 1-Butenoxid und/oder Propylenoxid, bevorzugt Ethylenoxid und/oder Propylenoxid, die in beliebiger Reihenfolge oder auch im Gemisch bei der Alkoxylierungsreaktion eingesetzt werden können.

Bei den Polyoxyalkylenmono-olen handelt es sich entweder um reine Polyethylenoxidpolyethermono-ole oder gemischte Polyalkylenoxidpolyethermono-ole, deren Alkylenoxideinheiten zu mindestens 30 mol-%, bevorzugt zu mindestens 50 mol-% aus Ethylenoxideinheiten bestehen. Besonders bevorzugt ist der Einsatz reiner Polyethylenoxidpolyethermono-ole.

Neben den OH-funktionellen ungesättigten Verbindungen der Komponente B) und den Polyoxyalkylenmono-olen C) können im erfindungsgemäßen Verfahren auch weitere Verbindungen D) eingesetzt werden, die verschieden von denen aus B) und/oder C) sind und NCO-reaktive Gruppen wie beispielsweise OH, SH oder NH aufweisen.

Dies können beispielsweise NH- oder SH-funktionelle Verbindungen mit unter Einwirkung aktinischer Strahlung mit ethylenisch ungesättigten Verbindungen unter Polymerisation reagierende Gruppen sein.

Auch unter Einwirkung von aktinischen Strahlen nichtreaktive Verbindungen, wie z.B. Polyetherpolyole, Polyesterpolyole, Polycarbonatpolyole und ein- oder mehrwertige Alkohole können zur Beeinflussung der Produkteigenschaften als Komponente D) mitverwendet werden. Als Polyole können niedermolekulare und in untergeordneter Menge auch höhermolekulare Hydroxylverbindungen eingesetzt werden.

Als niedermolekulare Polyole, die als Komponente D) mitverwendet werden können, können in der Polyurethan-Chemie übliche Polyole, mit Molekulargewichten von 62 bis 399, wie Ethylenglykol, Triethylenglykol, Tetraethylenglykol, Propandiol-1,2 und -1,3, Butandiol-1,4 und -1,3, Hexandiol-1,6, Octandiol-1,8, Neopentylglykol, 1,4-Bis(hydroxymethyl)cyclohexan, Bis(hydroxymethyl)tricyclo[5.2.1.0^{2.6}]decan oder 1,4-Bis(2-hydroxyethoxy)benzol, 2-Methyl-1,3-propandiol, 2,2,4-Trimethylpentandiol, 2-Ethyl-1,3-hexandiol, Dipropylenglykol, Polypropylenglykole, Dibutylenglykol, Polybutylenglykole, Bisphenol A, Tetrabrombisphenol A, Glycerin, Trimethylolpropan, Hexantriol-1,2,6, Butantriol-1,2,4, Pentaerythrit, Chinit, Mannit, Sorbit, Methylglykosid und 4,3,6-Dianhydrohexite verwendet werden.

Auch niedermolekulare Monohydroxyverbindungen können als Komponente D) eingesetzt werden, beispielhaft genannt seien gesättigte Monoalkohole wie Methanol, Ethanol, n-Propanol, Isopropanol, n-Butanol, Isobutanol, sec-Butanol, die Isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykol-monoalkylether wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol.

Höhermolekulare Hydroxylverbindungen als Komponente D) umfassen die in der Polyurethan-Chemie üblichen Hydroxypolyester, Hydroxypolyether, Hydroxypolythioether, Hydroxypolyacetale, Hydroxypolycarbonate, Dimerfettalkohole und/oder Esteramide, jeweils mit mittleren Molekulargewichten von 400 bis 8000 g/mol, bevorzugt solche mit mittleren Molekulargewichten von 500 bis 2 000 g/mol. Die Mitverwendung solcher höhermolekularen Hydroxylverbindungen ist aber nicht bevorzugt.

Als Verbindungen der optionalen Katalysatorkomponente E) kommen dem Fachmann an sich bekannte Urethanisierungskatalysatoren wie Organozinnverbindungen oder aminische Katalysatoren in Betracht. Als Organozinnverbindungen seien beispielhaft genannt: Dibutylzinndiacetat, Dibutylzinndilaurat, Dibutylzinn-bis-acetoacetonat und Zinncarboxylate wie beispielsweise Zinnoctoat. Die genannten Zinnkatalysatoren können gegebenenfalls in Kombination mit aminischen Katalysatoren wie Aminosilanen oder 1,4-Diazabicyclo[2.2.2]octan verwendet werden. Ebenfalls denkbar ist der Einsatz von Lewissauren Metallverbindungen, die Molybdän, Vanadium, Zirkonium, Cäsium, Bismuth oder Wolfram enthalten.

Bevorzugt wird in E) Dibutylzinndilaurat als Urethanisierungskatalysator eingesetzt.

Im erfindungsgemäßen Verfahren wird die Katalysatorkomponente E), sofern überhaupt mitverwendet, in Mengen von 0,001 - 5,0 Gew.-%, bevorzugt 0,001 - 0,1 Gew.-% und besonders bevorzugt 0,005 - 0,05 Gew.-% bezogen auf Festgehalt des Verfahrensprodukts eingesetzt.

Als Katalysator F) können dem Fachmann an sich bekannte Allophanatisierungskatalysatoren, wie beispielsweise die Zinksalze Zinkoctoat, Zinkacetylacetonat und Zink-2-ethylcaproat, Zirkoniumoctoat, Bismuthoctoat, Zinnoctoat oder Tetraalkylammoniumverbindungen, wie *N,N,N-*Trimethyl-*N-*2-hydroxypropyl-ammoniumhydroxid, *N,N,N-*Trimethyl-*N*-2-hydroxypropylammonium-2-ethylhexanoat oder Cholin-2-ethylhexanoat verwendet werden. Bevorzugt ist die Verwendung der Metalloctoate, besonders bevorzugt ist die Verwendung von Zinkoctoat.

Unter dem Begriff 'Zinkoctoat' werden im Rahmen dieser Erfindung auch technische Isomeren-Produktgemische verstanden, die neben verschiedenen isomeren Octoaten auch Anteile an Zink-Salzen von C₆-C₁₉-Fettsäuren enthalten können. Entsprechendes gilt auch für andere Metalloctoate.

Der Allophanatisierungskatalysator F) wird in Mengen von 0,001 - 5,0 Gew.-%, bevorzugt 0,001 - 0,1 Gew.-% und besonders bevorzugt 0,005 - 0,05 Gew.-% bezogen auf Festgehalt des Verfahrensprodukts eingesetzt.

Grundsätzlich kann schon für die Urethanisierungsreaktion in E) der Allophanatisierungskatalysator F) verwendet werden und die zweistufige Verfahrensweise zu einer einstufigen Umsetzung vereinfacht werden. Dies ist aber nicht bevorzugt, so dass der Allophanatisierungskatalysator erst dann zugesetzt wird, wenn die Urethangruppen ganz oder anteilig zu Allophanatgruppen umgesetzt werden sollen.

Der Katalysator F) kann in einer Portion auf einmal, portionsweise oder auch kontinuierlich zugegeben werden. Bevorzugt ist die Zugabe auf einmal.

Bei der Verwendung des bevorzugten Zinkoctoates als Allophanatisierungskatalysator F) kann die Allophanatisierungsreaktion gemäß der Lehre der EP-A 2 031 005 sehr langsam und oft unvollständig verlaufen, so dass es in diesem Fall bevorzugt ist, als Komponente G) ein tertiäres Amin einzusetzen. Geeignete tertiäre Amine G) besitzen vorzugsweise mindestens neun Kohlenstoffatome, wobei sowohl aromatische, wie auch aliphatische Reste enthalten sein können, die auch untereinander verbrückt sein können. Bevorzugt enthalten die Amine keine weiteren funktionellen Gruppen. Beispiele für geeignete Verbindungen sind *N,N,N-*Benzyldimethylamin, *N,N,N-*Dibenzylmethylamin, *N,N,N*-Cyclohexyldimethylamin, *N*-Methylmorpholin, *N,N,N-*Tribenzylamin, *N,N,N-*Tripropylamin, *N,N,N-*Tributylamin, *N,N,N*-Tripentylamin oder *N,N,N*-Trihexylamin. Bevorzugt ist dabei die Verwendung von *N,N,N-*Benzyldimethylamin.

Das tertiäre Amin, falls mitverwendet, wird in Mengen von 0,01 - 5,0 Gew.-%, bevorzugt 0,01 - 1,0 Gew.-% und besonders bevorzugt 0,05 - 0,5 Gew.-% bezogen auf Festgehalt des Verfahrensprodukts eingesetzt.

Bevorzugt wird die Reaktion der Allophanatisierung solange geführt, bis der NCO-Gehalt des Endproduktes unter 0,5 Gew.-%, besonders bevorzugt unter 0,1 Gew.-% liegt.

Es ist grundsätzlich möglich, einen Restgehalt an NCO-Gruppen mit NCO-reaktiven Verbindungen wie z.B. Alkoholen nach beendeter Allophanatisierungsreaktion umzusetzen. Dadurch werden Produkte mit ganz besonders niedrigen NCO-Gehalten erhalten.

Es ist auch möglich die Katalysatoren E) und/oder F) nach dem Fachmann bekannten Methoden auf Trägermaterialien zu bringen und als heterogene Katalysatoren zu verwenden.

Im erfindungsgemäßen Verfahren können optional an beliebiger Stelle Lösemittel oder Reaktivverdünner eingesetzt werden.

Komponente A) wird in Mengen von 20 bis 60 Gew.-%, bevorzugt von 30 bis 50 Gew.-%, besonders bevorzugt 35 bis 45 Gew.-%, Komponente B) in Mengen von 25 bis 50 Gew.-%, bevorzugt von 30 bis 45 Gew.-%, besonders bevorzugt von 35 bis 40 Gew.-%, Komponente C) in Mengen von 10 bis 35 Gew.-%, bevorzugt von 15 bis 25 Gew.-%, besonders bevorzugt von 19 bis 25 Gew.-%, Komponente D) in Mengen von 0 bis 40 Gew.-%, bevorzugt von 0 bis 15 Gew.-%, ganz besonders bevorzugt von 0 bis 5 Gew.-%, Komponente E) in Mengen von 0 bis 5 Gew.-%, bevorzugt von 0,001 bis 0,1 Gew.-%, besonders bevorzugt von 0,005 bis 0,05 Gew.-% , Komponente F) in Mengen von 0,001 bis 5 Gew.-%, bevorzugt von 0,001 bis 0,1 Gew.-%, ganz besonders bevorzugt von 0,005 bis 0,05 Gew-% eingesetzt und Komponente G) in Mengen von 0 bis 5 Gew.-%, bevorzugt von 0,01 bis 1 Gew.-%, ganz besonders bevorzugt von 0,05 bis 0,5 Gew-% eingesetzt mit der Maßgabe, dass die Summe der Gew.-% der Komponenten A) bis G) 100 beträgt.

Geeignete Lösemittel sind gegenüber den vorhandenen funktionellen Gruppen des Verfahrensprodukts vom Zeitpunkt der Zugabe bis zum Ende des Verfahrens inert. Geeignet sind z.B. in der Lacktechnik verwendete Lösemittel wie Kohlenwasserstoffe, Ketone und Ester, z.B. Toluol, Xylol, Isooctan, Aceton, Butanon, Methylisobutylketon, Ethylacetat, Butylacetat, Tetrahydrofuran, N-Methylpyrrolidon, Dimethylacetamid, Dimethylformamid, wobei jedoch bevorzugt kein Lösungsmittel zugesetzt wird.

Als Reaktivverdünner können Verbindungen mitverwendet werden, die bei der UV-Härtung ebenfalls (co)polymerisieren und somit in das Polymemetzwerk mit einbauen und gegenüber NCO-Gruppen inert sind. Solche Reaktivverdünner sind in P. K. T. Oldring (Ed.), Chemistry & Technology of UV & EB Formulations For Coatings, Inks & Paints, Vol. 2, 1991, SITA Technology, London, S. 237 - 285 exemplarisch beschrieben. Dies können Ester der Acrylsäure oder Methacrylsäure, bevorzugt der Acrylsäure mit mono- oder mehrfachfunktionellen Alkoholen sein. Als Alkohole eignen sich beispielsweise die isomeren Butanole, Pentanole, Hexanole, Heptanole, Octanole, Nonanole und Decanole, weiterhin cycloaliphatische Alkohole wie Isobornol, Cyclohexanol und alkylierte Cyclohexanole, Dicyclopentanol, arylaliphatischer Alkohole wie Phenoxyethanol und Nonylphenylethanol, sowie Tetrahydrofurfurylalkohole. Weiterhin können alkoxylierte Derivate dieser Alkohole verwendet werden. Geeignete zweiwertige Alkohole sind beispielsweise Alkohole wie Ethylenglykol, Propandiol-1,2, Propandiol-1,3, Diethylenglykol, Dipropylenglykol, die isomeren Butandiole, Neopentylglykol, Hexandiol-1,6, 2-Ethylhexandiol und Tripropylenglykol oder auch alkoxylierte Derivate dieser Alkohole. Bevorzugte zweiwertige Alkohole sind Hexandiol-1,6, Dipropylenglykol und Tripropylenglykol. Geeignete dreiwertige Alkohole sind Glycerin oder Trimethylolpropan oder deren alkoxylierte Derivate. Vierwertige Alkohole sind Pentaerythrit oder dessen alkoxylierte Derivate. Die Mitverwendung von Reaktivverdünnern ist aber nicht bevorzugt.

Das erfindungsgemäße Verfahren wird bei Temperaturen von höchstens 100°C, bevorzugt 20 bis 100°C, besonders bevorzugt von 40 bis 100°C, insbesondere bei 60 bis 90 °C durchgeführt.

Die erfindungsgemäßen Bindemittel können gegen vorzeitige Polymerisation stabilisiert werden. Daher gibt man vorzugsweise als Bestandteil einer oder mehrerer Komponenten (A), B), C), D), E) oder F)) vor und/oder während der Reaktion Stabilisatoren zu, die die Polymerisation inhibieren. Beispiele für geeignete Stabilisatoren sind z.B. Phenothiazin und Phenole wie para-Methoxyphenol, 2,5-Di-tert.-Butylhydrochinon oder 2,6-Di-tert.-butyl-4-methylphenol. Geeignet sind auch N-Oxyl-Verbindungen zur Stabilisierung wie z.B. 2,2,6,6-Tetramethylpiperidin-N-Oxid (TEMPO) oder seine Derivate. Ebenso können die Stabilisatoren auch chemisch mit in das Bindemittel eingebaut werden, dabei eignen sich Verbindungen der oben genannten Klassen insbesondere wenn sie noch weitere freie aliphatische Alkoholgruppen oder primäre oder sekundäre Amingruppen tragen und damit über Urethan- oder Harnstoff-Gruppen chemisch an Verbindungen der Komponente A) gebunden werden können. Hierfür besonders geeignet sind 2,2,6,6-Tetramethyl-4-hydroxypiperidin-N-Oxid.

Andere Stabilisatoren wie z. B. Verbindungen der Klasse der HALS (HALS = hindered amine light stabilizers) können ebenfalls verwendet werden, sind aber nicht bevorzugt.

Zur Stabilisierung der Reaktionsmischung, insbesondere der ungesättigten Gruppen gegen vorzeitige Polymerisation, kann ein sauerstoffhaltiges Gas, bevorzugt Luft, in und/oder über das Reaktionsgemisch geleitet werden. Es ist bevorzugt, dass das Gas einen möglichst geringen Anteil an Feuchtigkeit besitzt, um unerwünschte Reaktion bei Vorhandensein von Isocyanat zu verhindern.

Während der Herstellung der erfindungsgemäßen Bindemittel kann ein Stabilisator zugesetzt werden und abschließend, um eine Langzeitstabilität zu erreichen, nochmals mit einem phenolischen Stabilisator nachstabilisiert und ggf. mit Luft das Reaktionsprodukt gesättigt werden.

Es ist bevorzugt, im erfindungsgemäßen Verfahren die Stabilisatorkomponente in Mengen von 0,001 - 5,0 Gew.-%, bevorzugt 0,01 - 2,0 Gew.-% und besonders bevorzugt 0,05 - 1,0 Gew.-% bezogen auf Festgehalt des Verfahrensprodukts einzusetzen.

Bevorzugt wird das erfindungsgemäße Verfahren in einem Rührreaktor durchgeführt.

Der Verlauf der Reaktion kann durch geeignete im Reaktionsgefäß installierte Messgeräte und/oder anhand von Analysen an entnommenen Proben verfolgt werden. Geeignete Verfahren sind dem Fachmann bekannt. Es handelt sich beispielsweise um Viskositätsmessungen, Messungen des NCO-Gehalts, Messung des Brechungsindex, Messung des O H-Gehalts, Gaschromatographie (GC), kernmagnetische Resonanzspektroskopie (NMR), Infrarotspektroskopie (IR) und nahe Nahinfrarotspektroskopie (NIR). Bevorzugt ist die IR-Spektroskopie zur Kontrolle der vorhandenen freien NCO Gruppen (für aliphatische NCO-Gruppen liegt die Bande im IR-Spektrum bei ca. ν = 2272 cm⁻¹) und GC-Untersuchungen auf nicht umgesetzte Verbindungen aus A), B) C), und gegebenenfalls D).

In einer bevorzugten Ausführungsform wird die Urethanisierung der Komponenten A) bis D) gegebenenfalls in Gegenwart von E) durchgeführt. Nach Bestimmung des NCO-Gehalts wird ein NCO-Wert erreicht, der um bis zu 1,5 Gew.-% NCO (absolut), bevorzugt um bis zu 1,0 Gew.-% (absolut), besonders bevorzugt um bis zu 0,7 Gew.-% (absolut) vom theoretischen NCO-Gehalt.-% abweichen kann, und anschließend erfolgt durch Zugabe der Komponente E) und gegebenenfalls F), ohne weitere Zugabe von isocyanatgruppenhaltigen Verbindungen die Allophanantisierung.

Es ist grundsätzlich möglich, das erfindungsgemäße Verfahren in einer Stufe durchzuführen, wobei mit einem Katalysator oder einem Katalysatorgemisch, das sowohl die Urethanisierungs- als auch die Allophanatisierungsreaktion katalysiert, gearbeitet wird. Dabei werden Urethanisierung und Allophanatisierung parallel durchgeführt. Diese Verfahrensweise ist aber nicht bevorzugt.

Die nach dem erfindungsgemäßen Verfahren erhältlichen ungesättigten Allophanate, insbesondere die auf Basis des bevorzugt eingesetzten Hexamethylendiisocyanats, weisen bei einem Festkörpergehalt von 100% bevorzugt Scherviskositäten bei 23°C von ≤ 150 000 mPas, besonders bevorzugt ≤ 80 000 mPas und ganz besonders bevorzugt ≤ 50 000 mPas auf. Die Viskosität wird mit einem Kegel-Platte Rotationsviskosimeter, MCR 51 der Firma Anton-Paar, DE, mit einer Schergeschwindigkeit von 50 s-1 nach ISO/DIS 3219:1990 bestimmt.

Die erfindungsgemäßen strahlenhärtenden, wasseremulgierbaren Allophanate können zur Herstellung von Beschichtungen und Lacken sowie Klebstoffen, Druckfarben, Gießharzen, Dentalmassen, Schlichten, Photoresisten, Stereolithographiesystemen, Harzen für Verbundwerkstoffe und Dichtungsmassen verwendet werden. Im Falle der Verklebung oder Abdichtung ist allerdings Voraussetzung, dass bei Strahlenhärtung mindestens eines der beiden zu verklebenden oder miteinander abzudichtenden Substrate für die zur Härtung verwendete Strahlung durchlässig, also i. d. R. transparent sein muss. Bei Verwendung von Elektronenstrahlung zur Härtung ist auf eine hinreichende Durchlässigkeit für Elektronen zu achten. Bevorzugt ist die Verwendung der erfindungsgemäßen Allophanate als Bindemittel in Lacken und Beschichtungen.

Ein weiterer Gegenstand der Erfindung sind Beschichtungsmittel enthaltend
a) ein oder mehrere der erfindungsgemäßen strahlenhärtenden, wasseremulgierbaren Allophanate,
b) gegebenenfalls weitere von a) verschiedene Verbindungen, die unter Einwirkung aktinischer Strahlung mit ethylenisch ungesättigten Verbindungen unter Polymerisation reagierende Gruppen aufweisen,
c) gegebenenfalls weitere nicht strahlenhärtbare wässrige Bindemittel
d) Initiatoren,
e) gegebenenfalls Lösemittel und
f) gegebenenfalls Hilfs- und Zusatzstoffe.

Zu den Verbindungen der Komponente b) gehören nicht wässrige Verbindungen wie insbesondere Urethan(meth)acrylate bevorzugt auf Hexamethylendiisocyanat-, 1-Isocyanato-3,3,5-trimethyl-5-isocyanatomethyl-cyclohexan-, 4,4'-Diisocyanatodicyclohexylmethan- und/oder Trimethylhexamethylendiisocyanat-Basis, welche gegebenenfalls mit Isocyanurat-, Allophanat-, Biuret-, Uretdion- und/oder Iminooxadiazintriongruppen modifiziert sein können, welche keine gegenüber Isocyanatgruppen reaktiven Gruppen aufweisen.

Weiterhin können die bereits beschriebenen und in der Technik der strahlenhärtenden Beschichtungen bekannten Reaktivverdünner als Bestandteil von b) verwendet werden, sofern sie keine mit NCO-Gruppen reaktiven Gruppen enthalten.

Zu den Verbindungen der Komponente b) gehören auch in Wasser gelöste oder dispergierte Verbindungen wie insbesondere Dispersionen, die ungesättigte, strahlenhärtbare Gruppen enthalten, wie z. B. ungesättigte, strahlenhärtbare Gruppen enthaltende Dispersionen auf Polyester-, Polyurethan-, Polyepoxy(meth)acrylat-, Polyether-, Polyamid-, Polysiloxan-, Polycarbonat-, Polyepoxyacrylat-, Polyesteracrylat-, Polyurethan-Polyacrylat- und/oder Polyacrylatbasis. Die ungesättigten, strahlenhärtbaren Gruppen können dabei an einem der genannten Polymere gebunden vorliegen und/oder in Form von strahlenhärtbaren Monomeren, so genannten Reaktivverdünnern, dispergiert neben den genannten Polymeren vorliegen.

Zu den Verbindungen der Komponente c) gehören in Wasser gelöste oder dispergierte Verbindungen wie insbesondere Dispersionen, die keine ungesättigten, strahlenhärtbaren Gruppen enthalten, wie z. B. Dispersionen auf Polyester-, Polyurethan-, Polyether-, Polyamid-, Polysiloxan-, Polycarbonat-, Polyurethan-Polyacrylat- und /oder Polyacrylatbasis.

Insbesondere, wenn es sich bei den Komponenten b) und c) um in Wasser gelöste oder dispergierte Verbindungen wie insbesondere Dispersionen handelt, ist der Zusatz der erfindungsgemäßen wasseremulgierbaren, strahlenhärtbaren Allophanate a) vorteilhaft, da auf diese Weise der Festkörpergehalt der Komponenten b) und c) ohne wesentlichen Anstieg der resultierenden Viskosität angehoben werden kann.

Als Initiatoren der Komponente d) für eine radikalische Polymerisation können durch Strahlung und/oder thermisch aktivierbare Initiatoren zum Einsatz kommen. Fotoinitiatoren, die durch UV- oder sichtbares Licht aktiviert werden, sind hierbei bevorzugt. Bei den Fotoinitiatoren wird prinzipiell zwischen zwei Typen unterschieden, dem unimolekularen (Typ I) und dem bimolekularen (Typ II). Geeignete (Typ I)-Systeme sind aromatische Ketonverbindungen, wie z.B. Benzophenone in Kombination mit tertiären Aminen, Alkylbenzophenone, 4,4'-Bis(dimethylamino)benzophenon (Michlers Keton), Anthron und halogenierte Benzophenone oder Mischungen der genannten Typen. Weiter geeignet sind (Typ II)-Initiatoren wie Benzoin und seine Derivate, Benzilketale, Acylphosphinoxide z.B. 2,4,6-Trimethyl-benzoyl-diphenylphosphinoxid, Bisacylphosphinoxide, Phenylglyoxylsäureester, Campherchinon, α-Aminoalkylphenone, α,α-Dialkoxyacetophenone und α-Hydroxyalkylphenone.

Die Initiatoren, die in Mengen zwischen 0,1 und 10 Gew.-%, vorzugsweise 0,1 bis 5 Gew.-%, bezogen auf das Gewicht des Lackbindemittels, eingesetzt werden, können als einzelne Substanz oder, wegen häufiger vorteilhafter synergistischer Effekte, auch in Kombination miteinander verwendet werden.

Werden Elektronenstrahlen statt UV-Strahlung verwendet, so benötigt man keinen Fotoinitiator. Elektronenstrahlung wird wie dem Fachmann bekannt ist, mittels thermischer Emission erzeugt und über eine Potentialdifferenz beschleunigt. Die energiereichen Elektronen schlagen dann durch eine Titanfolie und werden auf die zu härtenden Beschichtungsmittel gelenkt. Die allgemeinen Prinzipien der Elektronenstrahlhärtung sind in "Chemistry & Technology of UV & EB Formulations for Coatings, Inks & Paints", Vol. 1, P K T Oldring (Ed.), SITA Technology, London, England, S. 101-157, 1991 im Detail beschrieben.

Im Falle einer thermischen Härtung der aktivierten Doppelbindungen, kann diese auch unter Zusatz von thermisch zerfallenden Radikalbildnern erfolgen. Geeignet sind, wie dem Fachmann bekannt ist, z.B. Peroxyverbindungen wie Dialkoxydicarbonate wie z.B. Bis(4-tert-butylcyclohexyl)peroxydicarbonat, Dialkylperoxide wie z.B. Dilaurylperoxid, Perester aromatischer oder aliphatischer Säuren wie z.B. tert.-Butylperbenzoat oder tert.-Amylperoxy 2-ethylhexanoat, anorganische Peroxide wie z. B. Ammoniumperoxodisulfat, Kaliumperoxodisulfat, organische Peroxide wie z.B. 2,2-Bis(tert.-butylperoxy)butan, Dicumylperoxid, tert.-Butylhydroperoxid oder auch Azoverbindungen wie 2,2'-Azobis[N-(2-propenyl)-2-methylpropionamid], 1-[(cyano-1-methylethyl)azo]formamid, 2,2'-Azobis(N-butyl-2-methylpropionamid), 2,2'-Azobis(N-cyclohexyl-2-methylpropionamid), 2,2'-Azobis{2-methyl-N-[2-(1-hydroxybutyl)]propionamid}, 2,2'-Azobis{2-methyl-N-[2-(1-hydroxybutyl)]propionamid, 2,2'-Azobis{2-methyl-N-[1,1-bis(hydroxymethyl)-2-hydroxyethyl] propionamid. Möglich sind auch hochsubstituierte 1,2-Diphenylethane (Benzpinakole), wie z. B. 3,4-Dimethyl-3,4-diphenylhexan, 1,1,2,2-Tetraphenyl-ethandiol-1,2 oder auch deren silylierten Derivate.

Es ist auch möglich eine Kombination von Fotoinitiatoren und thermisch aktivierbaren Initiatoren zu verwenden.

Als Komponente e) können optional auch organische Lösungsmittel mit verwendet werden, die dem Fachmann an sich bekannt sind. Es ist aber bevorzugt, Wasser als einziges Verdünnungsmittel einzusetzen.

Als Hilfs- und Zusatzstoffe (Komponente f) ) können zur Erhöhung der Wetterstabilität der gehärteten Lackschicht auch UV-Absorber und/oder HALS-Stabilisatoren enthalten sein. Bevorzugt ist eine Kombination von UV-Absorber und HALS-Stabilisatoren. Erstere weisen vorteilhaft einen Absorptionsbereich von maximal 390 nm auf, beispielsweise UV-Absorber wie Triphenyltriazintypen (z.B. Tinuvin^{®} 400 (Ciba Spezialitätenchemie GmbH, Lampertheim, DE)), Benztriazole (z.B. Tinuvin® 622 (Ciba Spezialitätenchemie GmbH, Lampertheim, DE)) oder Oxalsäuredianilide (z. B. Sanduvor^{®} 3206 (Clariant, Muttenz, CH) )) und werden in 0,5 - 3,5 Gew.-% bezogen auf Festharz zugegeben. Geeignete HALS-Stabilisatoren sind kommerziell erhältlich (z.B. Tinuvin^{®} 292 oder Tinuvin^{®} 123 (Ciba Spezialitätenchemie GmbH, Lampertheim, DE) oder Sanduvor^{®} 3258 (Clariant, Muttenz, CH)). Bevorzugte Mengen sind 0,5-2,5 Gew.-% bezogen auf Festharz.

Ebenfalls können in f) weitere in der Lacktechnologie bekannte Hilfs- und Zusatzstoffe wie z.B. Pigmente einschließlich Metallic-Effektpigmente, Farbstoffe, Mattierungsmittel, Füllstoffe, Verlaufs-, Benetzungs- und Entlüftungsadditive, Slip-Additive, Nanopartikel, Anti-Vergilbungsadditive, Verdicker und Additive zur Reduktion der Oberflächenspannung enthalten sein.

Das Auftragen der erfindungsgemäßen Beschichtungsmittel auf das zu beschichtende Material erfolgt mit den in der Beschichtungstechnologie üblichen und bekannten Methoden wie Spritzen, Rakeln, Walzen, Gießen, Tauchen, Schleudern, Streichen oder Sprühen oder durch Druck-Techniken wie Sieb-, Tief-, Flexo- oder Offsetdruck sowie durch Transfer-Methoden.

Geeignete Substrate sind beispielsweise Holz, Metall, insbesondere auch Metall wie es in den Anwendungen der sogenannten Draht-, Coil-, Can- oder Container-Lackierung verwendet wird, weiterhin Kunststoff auch in Form von Folien, insbesondere ABS, AMMA, ASA, CA, CAB, EP, UF, CF, MF, MPF, PF, PAN, PA, PE, HDPE, LDPE, LLDPE, UHMWPE, PET, PMMA, PP, PS, SB, PUR, PVC, RF, SAN, PBT, PPE, POM, PUR-RIM, SMC, BMC, PP-EPDM, und UP (Kurzbezeichnungen nach DIN 7728T1), Papier, Leder, Textilien, Filz, Glas, Holz, Holzwerkstoffe, Kork, anorganisch gebundene Substrate wie Holz- und Faserzementplatten, elektronische Baugruppen oder mineralische Untergründe. Es können auch Substrate, die aus verschiedenen der vorgenannten Materialien bestehen, oder bereits beschichtete Substrate wie Fahrzeuge, Flugzeuge oder Schiffe sowie deren Teile, insbesondere Karosserien oder Anbauteile lackiert werden. Es ist auch möglich die Beschichtungsmittel nur temporär auf ein Substrat aufzubringen, dann teilweise oder vollständig zu härten und gegebenenfalls wieder abzulösen, um z.B. Folien herzustellen.

Zur Aushärtung können durch Ablüften z.B. enthaltenes Wasser oder gegebenenfalls Lösemittel ganz oder teilweise entfernt werden.

Während des Ablüftens oder im Anschluss kann thermisch und/oder photochemisch ausgehärtet werden.

Falls notwendig kann die thermische Härtung bei Raumtemperatur aber auch bei erhöhter Temperatur, vorzugsweise bei 40 - 160 °C, bevorzugt bei 60 - 130 °C, besonders bevorzugt bei 80 - 110 °C erfolgen.

Bei Verwendung von Photoinitiatoren in d) erfolgt die Strahlungshärtung bevorzugt durch Einwirkung aktinischer Strahlung, beispielsweise durch Bestrahlung mit UV-Strahlung oder Tageslicht, z.B. Licht der Wellenlänge 200 bis 700 nm oder durch Bestrahlen mit energiereichen Elektronen (Elektronenstrahlung, 150 bis 300 keV). Als Strahlungsquellen für Licht oder UV-Strahlung dienen beispielsweise Hoch- oder Mitteldruckquecksilberdampflampen, wobei der Quecksilberdampf durch Dotierung mit anderen Elementen wie Gallium oder Eisen modifiziert sein kann. Laser, gepulste Lampen (unter der Bezeichnung UV-Blitzlichtstrahler bekannt), Halogenlampen oder Excimerstrahler sind ebenfalls möglich. Die Strahler können bauartbedingt oder durch Einsatz spezieller Filter und/oder Reflektoren so ausgestattet sein, das der Austritt eines Teils des UV-Spektrums verhindert wird. Beispielsweise kann z.B. aus arbeitshygienischen Gründen die dem UV-C oder UV-C und UV-B zugeordnete Strahlung herausgefiltert werden. Die Strahler können ortsunbeweglich installiert sein, so dass das zu bestrahlende Gut mittels einer mechanischen Vorrichtung an der Strahlungsquelle vorbeibewegt wird oder die Strahler können beweglich sein und das zu bestrahlende Gut verändert bei der Härtung seinen Ort nicht. Die üblicherweise zur Vernetzung ausreichende Strahlungsdosis bei UV-Härtung liegt im Bereich von 80 bis 5000 mJ/cm².

Die Bestrahlung kann gegebenenfalls auch unter Ausschluss von Sauerstoff, z. B. unter Inertgas-Atmosphäre oder Sauerstoff-reduzierter Atmosphäre durchgeführt werden. Als Inertgase eignen sich bevorzugt Stickstoff, Kohlendioxid, Edelgase oder Verbrennungsgase. Des Weiteren kann die Bestrahlung erfolgen, indem die Beschichtung mit für die Strahlung transparenten Medien abgedeckt wird. Beispiele hierfür sind z.B. Kunststofffolien, Glas oder Flüssigkeiten wie Wasser.

Je nach Strahlungsdosis und Aushärtungsbedingungen sind Typ und Konzentration des gegebenenfalls verwendeten Initiators in dem Fachmann bekannter Weise zu variieren.

Besonders bevorzugt werden zur Härtung Quecksilberhochdruckstrahler in ortsfesten Anlagen eingesetzt. Fotoinitiatoren werden dann in Konzentrationen von 0,1 bis 10 Gew.-%, besonders bevorzugt 0,2 bis 3,0 Gew.-% bezogen auf den Festkörper der Beschichtung eingesetzt. Zur Härtung dieser Beschichtungen wird bevorzugt eine Dosis von 200 bis 3000 mJ/cm² gemessen im Wellenlängenbereich von 200 bis 600 nm verwendet.

Bei Verwendung von thermisch aktivierbaren Initiatoren in d) erfolgt die Härtung durch Erhöhung der Temperatur. Die thermische Energie kann dabei durch Strahlung, Wärmeleitung und/oder Konvektion in die Beschichtung eingebracht werden, wobei üblicherweise die in der Beschichtungstechnologie gebräuchlichen Infrarot-Strahler, Nahinfrarotstrahler und/oder Öfen zum Einsatz kommen.

Es ist bevorzugt, die Aushärtung durch aktinische Strahlung vorzunehmen.

Die applizierten Schichtdicken (vor der Härtung) liegen typischerweise zwischen 0,5 und 5000 µm, bevorzugt zwischen 5 und 1000 µm, besonders bevorzugt zwischen 15 und 200 µm. Bei Verwendung von Lösungsmitteln wird dieses nach der Applikation und vor der Härtung durch die gängigen Methoden entfernt.

Ein weiterer Gegenstand der Erfindung ist ein Verfahren zur Herstellung von Beschichtungen von Substraten, dadurch gekennzeichnet, dass das erfindungsgemäße Beschichtungsmittel wie vorher beschrieben auf das Substrat aufgetragen und anschließend wie vorher beschrieben gehärtet wird.

Ein weiterer Gegenstand der Erfindung sind Substrate beschichtet mit den erfindungsgemäßen Beschichtungsmitteln, die die nach dem erfindungsgemäßen Verfahren hergestellte wasseremulgierbare Allophanate enthalten.

### Beispiele

Alle Prozentangaben beziehen sich sofern nicht abweichend angegeben auf Gewichtsprozent.

Der NCO-Gehalt wurde jeweils gemäß DIN EN ISO 11909 titrimetrisch verfolgt.

Die Viskositätsmessungen wurden mit einem Kegel-Platte Rotationsviskosimeter, MCR 51 der Firma Anton-Paar, DE, mit einer Schergeschwindigkeit von 50 s⁻¹ nach ISO/DIS 3219:1990 durchgeführt.

Die zur Zeit der Versuchsdurchführung herrschende Umgebungstemperatur von 23 °C wird als RT bezeichnet.

Die OH-Zahl wurde gemäß DIN 53240-2 bestimmt.

Der Festkörpergehalt wurde gravimetrisch nach Abdampfen aller flüchtigen Bestandteile gemäß DIN EN ISO 3251 bestimmt.

### Beispiel 1: (Verhältnis NCO/OH = 1,51:1,00)

In einem 2000 ml-Vierhals-Glaskolben mit Rückflusskühler, beheizbarem Ölbad, mechanischem Rührer, Luftdurchleitung (11/h), Innenthermometer und Tropftrichter wurden 340 g Hexamethylendiisocyanat und 100 mg Phenothiazin vorgelegt und auf 60 °C erwärmt. Es wurden 50 mg Dibutylzinndilaurat zugegeben und zunächst 372 g Hydroxypropylacrylat und dann 220 g Methoxypolyethylenglykol mit der Molmasse 750 g/mol (Pluriol® 750, BASF SE, Ludwigshafen, DE) so zugetropft, dass die Temperatur von 80 °C nicht überschritten wurde. Es wurde nachgerührt, bis der theoretische NCO-Wert von 6,8 % erreicht war. Dann wurden 3,38 g *N,N-*Dimethylbenzylamin zugegeben und etwa 5 Minuten gerührt, bis die Mischung homogenisiert war. Anschließend wurden 4,48 g Zinkoctoat (Borchi® Kat 22 der Firma Borchers GmbH, Langenfeld, DE) beigemengt und solange bei 80 °C gerührt, bis der NCO-Gehalt unter 0,2% gesunken war (etwa 16 Stunden). Es wurde ein farbloses Harz mit einer Viskosität von 18.500 mPas (23 °C) erhalten. Das Produkt war bei einem Festkörpergehalt von ca. 40% gut wasseremulgierbar. Die Emulsion war über mehrere Tage stabil.

### Beispiel 2: (Verhältnis NCO/OH = 1,51:1,00)

In einem 2000 ml-Vierhals-Glaskolben mit Rückflusskühler, beheizbarem Ölbad, mechanischem Rührer, Luftdurchleitung (11/h), Innenthermometer und Tropftrichter wurden 336 g Hexamethylendiisocyanat und 80 mg Phenothiazin vorgelegt und auf 60 °C erwärmt. Es wurden 40 mg Dibutylzinndilaurat zugegeben und nacheinander 297 g Hydroxypropylacrylat, 121 g Methoxypolyethylenglykol mit der Molmasse 750 g/mol (Pluriol® 750, BASF SE, Ludwigshafen, DE) und dann 20,0 g Cyclohexanol so zugetropft, dass die Temperatur von 80 °C nicht überschritten wurde. Es wurde nachgerührt, bis der theoretische NCO-Wert von 7,3% erreicht war. Dann wurden 2,84 g *N,N-*Dimethylbenzylamin zugegeben und etwa 5 Minuten gerührt, bis die Mischung homogenisiert war. Anschließend wurden 3,76 g Zinkoctoat (Borchi® Kat 22 der Firma Borchers GmbH, Langenfeld, DE) beigemengt und solange bei 80 °C gerührt, bis der NCO-Gehalt unter 0,2% gesunken war (etwa 16 Stunden). Es wurde ein farbloses Harz mit einer Viskosität von 30.000 mPas (23 °C) erhalten. Das Produkt war bei einem Festkörpergehalt von ca. 40% gut wasseremulgierbar. Die Emulsion war über mehrere Tage stabil.

### Beispiel 3: (Verhältnis NCO/OH = 1,51:1,00)

In einem 2000 ml-Vierhals-Glaskolben mit Rückflusskühler, beheizbarem Ölbad, mechanischem Rührer, Luftdurchleitung (11/h), Innenthermometer und Tropftrichter wurden 336 g Hexamethylendiisocyanat und 80 mg Phenothiazin vorgelegt und auf 60 °C erwärmt. **Es** wurden 40 mg Dibutylzinndilaurat zugegeben und nacheinander 177 g Hydroxyethylacrylat, 99 g Hydroxypropylacrylat, 125 g Methoxypolyethylenglykol mit der Molmasse 750 g/mol (Pluriol® 750, BASF SE, Ludwigshafen, DE) und dann 20,0 g Cyclohexanol so zugetropft, dass die Temperatur von 80 °C nicht überschritten wurde. Es wurde nachgerührt, bis der theoretische NCO-Wert von 7,5% erreicht war. Dann wurden 2,84 g *N,N-*Dimethylbenzylamin zugegeben und etwa 5 Minuten gerührt, bis die Mischung homogenisiert war. Anschließend wurden 3,76 g Zinkoctoat (Borchi® Kat 22 der Firma Borchers GmbH, Langenfeld, DE) beigemengt und solange bei 80 °C gerührt, bis der NCO-Gehalt unter 0,2% gesunken war (etwa 16 Stunden). Es wurde ein farbloses Harz mit einer Viskosität von 17.600 mPas (23 °C) erhalten. Das Produkt war bei einem Festkörpergehalt von ca. 40% gut wasseremulgierbar. Die Emulsion war über mehrere Tage stabil.

### Beispiel 4 (Vergleich): (Verhältnis NCO/OH = 1,42:1,00)

In einem 2000 ml-Vierhals-Glaskolben mit Rückflusskühler, beheizbarem Ölbad, mechanischem Rührer, Luftdurchleitung (11/h), Innenthermometer und Tropftrichter wurden 355 g Hexamethylendiisocyanat und 100 mg Phenothiazin vorgelegt und auf 60 °C erwärmt. Es wurden 50 mg Dibutylzinndilaurat zugegeben und zunächst 262 g Hydroxypropylacrylat, dann 61 g Hydroxyethylacrylat und schließlich 313 g Methoxypolyethylenglykol mit der Molmasse 750 g/mol (Pluriol® 750, BASF SE, Ludwigshafen, DE) so zugetropft, dass die Temperatur von 80 °C nicht überschritten wurde. Es wurde nachgerührt, bis der theoretische NCO-Wert von 5,38% erreicht war. Dann wurden 3,01 g *N,N-*Dimethylbenzylamin zugegeben und etwa 5 Minuten gerührt, bis die Mischung homogenisiert war. Anschließend wurden 4,48 g Zinkoctoat (Borchi® Kat 22 der Firma Borchers GmbH, Langenfeld, DE) beigemengt und solange bei 80 °C gerührt, bis der NCO-Gehalt unter 0,2% gesunken war (etwa 16 Stunden). Es wurde ein farbloses Harz mit einer Viskosität von 6.640 mPas (23 °C) erhalten. Das Produkt ließ sich bei einem Festkörpergehalt von ca. 40% nur schwer in Wasser emulgieren und trennte sich nach kurzer Zeit wieder in zwei Phasen auf.

Es zeigt sich, dass ein höheres NCO/OH-Verhältnis (was zu einem höheren Molekulargewicht führt) nötig ist, um zu stabilen Emulsionen zu kommen.

## Patentansprüche

1. Verfahren zur Herstellung von wasseremulgierbaren, strahlenhärtenden Allophanaten mit Restmonomergehalten von weniger als 0,5 Gew.-% und einem NCO-Gehalt von weniger als 1 Gew.-%, bei dem aus
A) mindestens einer isocyanatgruppenhaltigen Verbindung,
B) mindestens einer hydroxyfunktionellen Verbindung, die unter Einwirkung aktinischer Strahlung mit ethylenisch ungesättigten Verbindungen unter Polymerisation reagierende Gruppen (strahlenhärtende Gruppen) aufweist,
C) mindestens einem Polyoxyalkylenmono-ol
E) gegebenenfalls in Anwesenheit eines Katalysators
NCO-gruppenhaltige Urethane mit strahlenhärtenden Gruppen gebildet werden, die anschließend ohne weitere Zugabe an isocyanatgruppenhaltigen Verbindungen in Anwesenheit
F) eines Allophanatisierungskatalysators und
G) gegebenenfalls eines tertiäten Amins
umgesetzt werden,
wobei das Verhältnis von NCO-Gruppen der Verbindungen aus A) zu den OH-Gruppen von den Verbindungen aus B) und C) 1,80 : 1,0 bis 1,46 : 1,0, beträgt.

2. Verfahren gemäß Anspruch 1, **dadurch gekennzeichnet, dass** als Komponente D) mindestens eine von B) und/oder C) verschiedene Verbindung mit NCO-reaktiven Gruppen, eingesetzt wird, wobei das Verhältnis von NCO-Gruppen der Verbindungen aus A) zu den OH-Gruppen von den Verbindungen aus B), C) und D) 1,80 : 1,0 bis 1,46 : 1,0, beträgt.

3. Verfahren gemäß Anspruch 1 oder 2, **dadurch gekennzeichnet, dass** in Komponente A) Hexamethylendiisocyanat (HDI), Isophorondiisocyanat (IPDI) und/oder 4,4'-Diisocyanatodicyclohexylmethan eingesetzt werden.

4. Verfahren gemäß einem der Ansprüche 1 bis 3, **dadurch gekennzeichnet, dass** das Verhältnis von NCO-Gruppen der Verbindungen aus A) zu den OH-Gruppen von den Verbindungen aus B), C) und D) 1,7 : 1,0 bis 1,47 : 1,0, beträgt.

5. Verfahren gemäß einem der Ansprüche 1 bis 4, **dadurch gekennzeichnet, dass** das Verhältnis von NCO-Gruppen der Verbindungen aus A) zu den OH-Gruppen von den Verbindungen aus B), C) und D) 1,65 : 1,0 bis 1,48 : 1,0, beträgt.

6. Verfahren gemäß einem der Ansprüche 1 bis 5, **dadurch gekennzeichnet, dass** in C) Polyoxyalkylenmono-ole eingesetzt werden, die einen Anteil von 30 bis 100 Gew.-% an Bausteinen, die vom Ethylenoxid abgeleitet sind, enthalten.

7. Verfahren gemäß einem der Ansprüche 1 bis 6, **dadurch gekennzeichnet, dass** in Komponente B) Hydroxyethyl(meth)acrylat und/oder Hydroxypropyl(meth)acrylat eingesetzt werden.

8. Verfahren gemäß einem der Ansprüche 1 bis 7, **dadurch gekennzeichnet, dass** die Allophanatisierung solange geführt wird, bis das Endprodukt einen NCO-Gehalt von unter 0,1 Gew.-% aufweist.

9. Verfahren gemäß einem der Ansprüche 1 bis 8, **dadurch gekennzeichnet, dass** Komponente A) in Mengen von 20 bis 60 Gew.-%, Komponente B) in Mengen von 25 bis 50 Gew.-%, Komponente C) in Mengen von 10 bis 35 Gew.-%, Komponente D) in Mengen von 0 bis 40 Gew.-%, Komponente E) in Mengen von 0 bis 5 Gew.-%, Komponente F) in Mengen von 0,001 bis 5 Gew.-%, und Komponente G) in Mengen von 0 bis 5 Gew.-%, eingesetzt werden, mit der Maßgabe, dass die Summe der Gew.-% der Komponenten A) bis G) 100 beträgt.

10. Verfahren gemäß einem der Ansprüche 1 bis 9, **dadurch gekennzeichnet, dass** die Urethanisierung der Komponenten A) bis D) gegebenenfalls in Gegenwart von E) durchgeführt wird, nach Bestimmung des NCO-Gehalts ein NCO-Wert erreicht wird, der um bis zu 1,5 Gew.-% NCO (absolut) vom theoretischen NCO-Gehalt.-% abweicht, und anschließend durch Zugabe der Komponente E) und gegebenenfalls F), ohne weitere Zugabe von isocyanatgruppenhaltigen Verbindungen die Allophanantisierung erfolgt.

11. Strahlenhärtende, wasseremulgierbare Allophanate erhältlich nach einem Verfahren gemäß einem der Ansprüche 1 bis 10.

12. Verwendung der strahlenhärtenden, wasseremulgierbaren Allophanate gemäß Anspruch 11 bei der Herstellung von Lacken sowie Klebstoffen, Druckfarben, Gießharzen, Dentalmassen, Schlichten, Photoresisten, Stereolithographiesystemen, Harzen für Verbundwerkstoffe und Dichtungsmassen.

13. Beschichtungsmittel enthaltend
a) ein oder mehrere strahlenhärtende, wasseremulgierbare Allophanate gemäß Anspruch 11,
b) gegebenenfalls weitere von a) verschiedene Verbindungen, die unter Einwirkung aktinischer Strahlung mit ethylenisch ungesättigten Verbindungen unter Polymerisation reagierende Gruppen aufweisen,
c) gegebenenfalls weitere nicht strahlenhärtbare wässrige Bindemittel
d) Initiatoren,
e) gegebenenfalls Lösemittel und
f) gegebenenfalls Hilfs- und Zusatzstoffe.

14. Substrate beschichtet mit den Beschichtungsmitteln gemäß Anspruch 13.

## Claims

1. Process for preparing water-emulsifiable, radiation-curing allophanates having residual monomer contents of less than 0.5 weight% and an NCO content of less than 1 weight%, in which
A) at least one compound containing isocyanate groups,
B) at least one hydroxy-functional compound which has groups which undergo polymerization reaction with ethylenically unsaturated compounds on exposure to actinic radiation (radiation-curing groups),
C) at least one polyoxyalkylene mono-ol
E) optionally in the presence of a catalyst are used to form NCO-group-containing urethanes having radiation-curing groups, which are subsequently reacted, without further addition of compounds containing isocyanate groups in the presence
F) of an allophanatization catalyst and
G) optionally of a tertiary amine,
the ratio of NCO groups of the compounds from A) to the OH groups of the compounds from B) and C) being 1.80:1.0 to 1.46:1.0.

2. Process according to Claim 1, **characterized in that** use is made as component D) of at least one compound which is different from B) and/or C) and which has NCO reactive groups, the ratio of NCO groups of the compounds from A) to the OH groups of the compounds from B), C) and D) being 1.80:1.0 to 1.46:1.0.

3. Process according to Claim 1 or 2, **characterized in that** hexamethylene diisocyanate (HDI), isophorone diisocyanate (IPDI) and/or 4,4'-diiso-cyanatodicyclohexylmethane are used in component A).

4. Process according to any of Claims 1 to 3, **characterized in that** the ratio of NCO groups of the compounds from A) to the OH groups of the compounds from B), C) and D) is 1.7:1.0 to 1.47:1.0.

5. Process according to any of Claims 1 to 4, **characterized in that** the ratio of NCO groups of the compounds from A) to the OH groups of the compounds from B), C) and D) is 1.65:1.0 to 1.48:1.0.

6. Process according to any of Claims 1 to 5, **characterized in that** polyoxyalkylene mono-ols are used in C) which include a fraction of 30 to 100 weight% of units derived from ethylene oxide.

7. Process according to any of Claims 1 to 6, **characterized in that** hydroxyethyl (meth)acrylate and/or hydroxypropyl (meth)acrylate are used in component B).

8. Process according to any of Claims 1 to 7, **characterized in that** the allophanatization is continued until the end product has an NCO content of less than 0.1 weight%.

9. Process according to any of Claims 1 to 8, **characterized in that** component A) is used in amounts of 20 to 60 weight%, component B) in amounts of 25 to 50 weight%, component C) in amounts of 10 to 35 weight%, component D) in amounts of 0 to 40 weight%, component E) in amounts of 0 to 5 weight%, component F) in amounts of 0.001 to 5 weight%, and component G) in amounts of 0 to 5 weight%, with the proviso that the sum of the weight% of components A) to G) is 100.

10. Process according to any of Claims 1 to 9, **characterized in that** the urethanization of components A) to D) is carried out optionally in the presence of E); following determination of the NCO content, an NCO value is attained which deviates by up to 1.5 weight% NCO (absolute) from the theoretical NCO content%; and subsequently, by addition of component E) and optionally F), without further addition of compounds containing isocyanate groups, the allophanatization takes place.

11. Radiation-curing, water-emulsifiable allophanates obtainable by a process according to any of Claims 1 to 10.

12. Use of the radiation-curing, water-emulsifiable allophanates according to Claim 11 in the production of paints and also adhesives, printing inks, casting resins, dental compositions, sizes, photoresists, stereolithography systems, resins for composite materials, and sealants.

13. Coating compositions comprising
a) one or more radiation-curing, water-emulsifiable allophanates according to Claim 11,
b) optionally further compounds, different from a), which have groups which undergo polymerization reaction with ethylenically unsaturated compounds on exposure to actinic radiation,
c) optionally further aqueous binders, which are not radiation-curable,
d) initiators,
e) optionally solvents, and
f) optionally auxiliaries and adjuvants.

14. Substrates coated with the coating compositions according to Claim 13.

## Revendications

1. Procédé pour la préparation d'allophanates émulsifiables dans l'eau, durcissant sous l'effet de rayons, présentant des teneurs résiduelles en monomères inférieures à 0,5% en poids et une teneur en NCO inférieure à 1% en poids, dans lequel on forme, à partir
A) d'au moins un composé contenant des groupes isocyanate,
B) d'au moins un composé à fonctionnalité hydroxy, qui présente des groupes réagissant avec polymérisation sous l'effet d'un rayonnement actinique avec des composés éthyléniquement insaturés (groupes durcissant sous l'effet de rayons) et
C) d'au moins un polyoxyalkylène-mono-ol,
E) le cas échéant en présence d'un catalyseur des uréthanes contenant des groupes NCO présentant des groupes durcissant sous l'effet de rayons, qui sont ensuite transformés, sans autre addition de composés contenant des groupes isocyanate, en présence
F) d'un catalyseur de transformation en allophanates et
G) le cas échéant d'une amine tertiaire,
le rapport des groupes NCO des composés de A) aux groupes OH des composés de B) et de C) étant de 1,80:1,0 à 1,46:1,0.

2. Procédé selon la revendication 1, **caractérisé en ce qu'**on utilise, comme composant D), au moins un composé différent de B) et/ou de C), présentant des groupes réactifs avec NCO, le rapport des groupes NCO des composés de A) aux groupes OH des composants de B), C) et D) valant 1,80:1,0 à 1,46:1,0.

3. Procédé selon la revendication 1 ou 2, **caractérisé en ce qu'**on utilise, dans le composant A), de l'hexaméthylènediisocyanate (HDI), de l'isophoronediisocyanate (IPDI) et/ou du 4,4'-diisocyanatodicyclohexylméthane.

4. Procédé selon l'une quelconque des revendications 1 à 3, **caractérisé en ce que** le rapport des groupes NCO des composés de A) aux groupes OH des composés de B), C) et D) est de 1,7:1,0 à 1,47:1,0.

5. Procédé selon l'une quelconque des revendications 1 à 4, **caractérisé en ce que** le rapport des groupes NCO des composés de A) aux groupes OH des composés de B), C) et D) est de 1,65:1,0 à 1,48:1,0.

6. Procédé selon l'une quelconque des revendications 1 à 5, **caractérisé en ce qu'**on utilise, dans C), des polyoxyalkylène-mono-ols qui contiennent une proportion de 30 à 100% en poids d'éléments qui sont dérivés d'oxyde d'éthylène.

7. Procédé selon l'une quelconque des revendications 1 à 6, **caractérisé en ce qu'**on utilise, dans le composant B), du (méth)acrylate d'hydroxyéthyle et/ou du (méth)acrylate d'hydroxypropyle.

8. Procédé selon l'une quelconque des revendications 1 à 7, **caractérisé en ce que** la transformation en allophanates est réalisée jusqu'à ce que le produit fini présente une teneur en NCO inférieure à 0,1% en poids.

9. Procédé selon l'une quelconque des revendications 1 à 8, **caractérisé en ce que** le composant A) est utilisé en des quantités de 20 à 60% en poids, le composant B) est utilisé en des quantités de 25 à 50% en poids, le composant C) est utilisé en des quantités de 10 à 35% en poids, le composant D) est utilisé en des quantités de 0 à 40% en poids, le composant E) est utilisé en des quantités de 0 à 5% en poids, le composant F) est utilisé en des quantités de 0,001 à 5% en poids et le composant G) est utilisé en des quantités de 0 à 5% en poids, à condition que la somme des % en poids des composant A) à G) vaille 100.

10. Procédé selon l'une quelconque des revendications 1 à 9, **caractérisé en ce que** l'uréthanisation des composants A) à D) est réalisée, le cas échéant, en présence de E), on obtient une valeur NCO, selon la détermination de la teneur en NCO, qui s'écarte de jusqu'à 1,5% en poids de NCO (absolu) de la teneur théorique en % de NCO et la transformation en allophanates a lieu ensuite par addition du composant E) et le cas échéant F), sans autre addition de composés contenant des groupes isocyanate.

11. Allophanates émulsifiables dans l'eau, durcissant sous l'effet de rayons, pouvant être obtenus selon un procédé selon l'une quelconque des revendications 1 à 10.

12. Utilisation des allophanates émulsifiables dans l'eau, durcissant sous l'effet de rayons selon la revendication 11 lors de la préparation de laques ainsi que d'adhésifs, d'encres d'imprimerie, de résines coulées, de masses dentaires, de barbotines, de résines photosensibles, de systèmes de stéréolithographie, de résines pour matériaux composites et de masses d'étanchéité.

13. Agents de revêtement, contenant
a) un ou plusieurs allophanates émulsifiables dans l'eau, durcissant sous l'effet de rayons selon la revendication 11,
b) le cas échéant d'autres composés différents de ceux de a), qui présentent des groupes réagissant avec polymérisation sous l'effet d'un rayonnement actinique avec des composés éthyléniquement insaturés,
c) le cas échéant d'autres liants aqueux ne durcissant pas sous l'effet de rayons,
d) des initiateurs,
e) le cas échéant des solvants et
f) le cas échéant des adjuvants et des additifs.

14. Substrats revêtus par les agents de revêtement selon la revendication 13.
